# FASCICULE DE BREVET EUROPEEN

(11) **EP 1 607 074 B1**
(45) Date de publication et mention de la délivrance du brevet: **11.03.2009**
(21) Numéro de dépôt: 05291272.2
(22) Date de dépôt: 14.06.2005
(51) Int. Cl.: A61F 7/10

(54) **Compresse à effet refroidissant sous présentation stérile**
Sterile Kühlkompresse
Sterile cooling compress

(30) Priorité: 18.06.2004 FR 0406633
(43) Date de publication de la demande: 21.12.2005
(73) Titulaire: Caceres, Patrick, 69110 Sainte Foy-les-Lyon (FR); Caceres, Franck, 69003 Lyon (FR)
(72) Inventeur: Caceres, Patrick, 69110 Sainte Foy-les-Lyon (FR); Caceres, Franck, 69003 Lyon (FR)
(74) Mandataire: Thibon-Littaye, Annick

(56) Documents cités:
- WO-A-03/055425
- US-A- 3 893 834
- US-A- 5 447 532
- US-A- 5 800 483
- US-A1- 2004 024 438
- US-B1- 6 524 331

## Description

La présente invention concerne la conception et la réalisation d'une compresse sous présentation stérile, qui présente, après activation, un effet refroidissant.

On entend par compresse tout type de pansement, bandage ou article similaire, qui est destiné à être appliqué sur le corps pour une opération de soin, médical ou autre.

La compresse suivant l'invention est destinée à être utilisée pour le soin par cryothermie. On connaît depuis longtemps l'efficacité du froid pour le soin de certaines affections physiques, et notamment pour soulager la douleur ou pour provoquer le dégonflement d'oedèmes. Dans certains cas, il est nécessaire que le froid soit appliqué sur la partie du corps à traiter en respectant des conditions de stérilité. On pense notamment au cas des plaies ou des zones où la peau est irritée, qui sont susceptibles de s'infecter au contact d'agents microbiens. Dans le cas du soin de telles plaies, il est indispensable que tout élément mis au contact de la plaie soit stérile.

La compresse suivant l'invention peut être utilisée dans tout endroit où il peut être souhaitable de délivrer du froid au corps, qu'il soit humain ou animal, dans des conditions de stérilité. Elle est particulièrement avantageuse en milieu hospitalier, puisque dans un tel milieu il est de rigueur d'utiliser uniquement du matériel stérile, pour éviter au maximum la propagation des infections, et notamment des infections nosocomiales.

On connaît de l'art antérieur des compresses à effet rafraîchissant destinées à être appliquées sur le corps. De telles compresses, tout à fait avantageuses du point de vue de l'efficacité du froid délivré et de leur confort d'utilisation, ont notamment été conçues à partir de polymères à forte capacité d'absorption d'eau, qui produisent du froid par évaporation et désorption de l'eau absorbée. On peut citer à titre d'exemple les compresses décrites dans le document US 5 597 577. Ces compresses sont composées de particules de polymère absorbant incluses dans une enveloppe textile. Afin d'exercer leur action de refroidissement, elles doivent tout d'abord être activées, c'est-à-dire immergées dans l'eau, afin que les particules de polymère gonflent par absorption d'eau. Elles exercent ensuite leur effet rafraîchissant, par évaporation et désorption de l'eau absorbée par le polymère. De telles compresses sont tout à fait adaptées pour une utilisation courante chez les particuliers, pour soulager la douleur due par exemple à une blessure ligamentaire ou musculaire, ou même à une migraine. Cependant, elles ne conviennent pas pour une utilisation en milieu hospitalier par exemple, puisqu'elles ne se présentent pas sous une forme stérile, et qu'il est en outre impossible de les activer dans des conditions de stérilité satisfaisantes.

Le document US 5 447 532 propose, afin d'activer une compresse rafraîchissante à base d'un polymère absorbant, de la disposer à l'intérieur d'un sac en plastique, puis de verser à l'intérieur de ce sac l'eau nécessaire à son activation. Le sac est ensuite fermé durant le temps nécessaire à l'absorption de l'eau par la compresse. Ce procédé d'activation ne permet pas d'obtenir une compresse stérile. Le problème de la stérilité de la compresse n'est pas abordé dans ce document.

L'invention vise à remédier à ces inconvénients, en proposant un système de préparation d'une compresse à base de polymère absorbant, qui préserve l'état stérile des éléments qui le composent. Cette compresse peut ainsi être utilisée en toute sécurité, notamment en milieu hospitalier. Appliquée sur la partie du corps à soulager, elle y délivre du froid pendant un temps relativement long.

La compresse à effet refroidissant choisie dans le cadre de l'invention est à base de particules d'un polymère à forte capacité d'absorption d'eau. Les compresses de ce type, qui sont connues de l'art antérieur, présentent une bonne inertie à la reprise en température, et donc une bonne efficacité de délivrance de froid. Leur principe de fonctionnement est bien connu de l'art antérieur. On pourra notamment se référer pour cela au document US 5 597 577 précité.

La compresse suivant l'invention se présente incluse avec une réserve d'eau pour son activation dans un article qui comporte, à l'intérieur d'un sachet extérieur étanche à l'eau, les deux éléments suivants : d'une part, une poche intérieure remplie d'eau et fermée étanche à l'eau par une paroi comportant une zone frangible ; et d'autre part, la compresse réalisée sous forme d'une enveloppe au moins en partie perméable à l'eau contenant les particules de polymère à l'état sec. Le sachet est constitué en une matière de nature à maintenir la poche et la compresse à l'état stérile. Il est fermé de façon étanche à l'eau et aux germes microbiens. La poche contenant l'eau et les différents éléments constituant la compresse sont réalisés en des matériaux qui sont compatibles avec un traitement de stérilisation.

L'enveloppe de la compresse qui contient les particules de polymère absorbant d'eau est avantageusement conçue de telle sorte que lorsque les particules sont à l'état sec, il reste dans l'enveloppe un volume suffisant pour permettre aux particules de gonfler par absorption d'eau. Ainsi, lorsque les particules sont à l'état sec, la compresse se présente sous une forme aplatie, les parois opposées de l'enveloppe étant effondrées l'une contre l'autre. Par contre, lorsque les particules contenues dans l'enveloppe absorbent de l'eau, elles se dilatent sous l'effet de cette absorption, et la compresse se présente alors sous une forme gonflée, avec une épaisseur de l'ordre du centimètre. Lorsque la compresse est appliquée sur le corps d'un individu, l'eau contenue dans les particules se réchauffe et se vaporise. Elle est alors désorbée des particules, en même temps qu'un effet de refroidissement est créé.

Les différents constituants de l'article suivant l'invention sont choisis compatibles avec un traitement de stérilisation. Suivant un mode de réalisation préféré de l'invention, le sachet extérieur, la poche intérieure et la compresse ont en particulier la particularité d'être compatibles avec un traitement de stérilisation par rayons gamma, notamment à 20 à 50 kGy, et/ou avec un traitement de stérilisation par rayons bêta.

On assure ainsi avantageusement que l'article suivant l'invention, c'est-à-dire le sachet extérieur contenant la compresse et la poche remplie d'eau, puisse être soumis à un traitement de stérilisation. De plus, le matériau constituant les parois du sachet extérieur est de nature à maintenir la poche et la compresse à l'état stérile. Ce matériau constitue notamment une barrière anti-microbienne, qui empêche toute contamination extérieure au sachet de pénétrer dans ce dernier. Ainsi, une fois le traitement de stérilisation réalisé, l'article suivant l'invention peut être conservé en toute sécurité : les éléments qu'il contient, notamment la compresse et la poche d'eau, y sont maintenus dans un état stérile.

Afin de pouvoir utiliser la compresse pour son effet refroidissant, il est nécessaire de l'activer. Dans le principe, comme il a été expliqué ci-avant dans la présente description, ceci est réalisé en immergeant la compresse dans l'eau, de manière à ce que les particules de polymère gonflent par absorption d'eau, puis en plaçant la compresse pendant quelques heures au congélateur. Cette dernière étape a pour objectif de faire diminuer la température de l'eau contenue dans les particules, et de la faire passer à l'état solide. Ainsi, lorsque la compresse est ensuite appliquée sur le corps, l'eau, avant de se vaporiser, doit préalablement se liquéfier. II en résulte une plus grande inertie de remontée en température de la compresse.

Avec l'article suivant l'invention, et en cela la présente invention s'avère tout à fait avantageuse, l'activation de la compresse est effectuée dans des conditions stériles, et de façon très simple. Une pression est exercée, à travers le sachet extérieur, sur la poche intérieure, de manière à faire rompre la zone frangible de cette dernière. L'article suivant l'invention est avantageusement conçu de telle sorte que lors de cette opération il n'y a aucun risque que ce soit le sachet extérieur qui cède et qui s'ouvre sous l'effet de cette pression. Ainsi, suivant une caractéristique avantageuse de l'invention, le sachet extérieur est fermé d'une façon résistante, et de telle sorte qu'il faut, pour l'ouvrir, exercer une pression bien plus importante que celle qui est nécessaire pour provoquer la rupture de la zone frangible de la poche intérieure.

L'eau se libère hors de la poche intérieure, elle se répand dans le sachet, elle vient au contact de la compresse, elle traverse l'enveloppe perméable à l'eau et elle est absorbée, et retenue, par les particules de polymère. Une fois que la compresse est gonflée d'eau, l'article dans son entier peut être placé au froid, notamment au congélateur, pendant un temps suffisant pour permettre à l'eau de refroidir. Dans toutes ces étapes, le sachet extérieur n'est jamais ouvert, si bien que la compresse, et l'eau qu'elle absorbe, se trouvent en permanence dans un environnement stérile.

Enfin, au moment souhaité pour l'utilisation, le sachet extérieur est ouvert, en respectant de préférence des conditions sanitaires optimales (port de gants et utilisation de pinces stériles par exemple), et la compresse en est extraite. Elle fournit alors un effet de refroidissement efficace.

Suivant une caractéristique avantageuse de l'invention, la poche intérieure contient un volume d'eau sensiblement égal, et de préférence légèrement inférieur, au volume disponible dans l'enveloppe pour permettre le gonflement des particules par absorption d'eau. On ne tient pas compte pour évaluer ce volume de la capacité de dilatation propre des parois de l'enveloppe lorsque les particules de polymère gonflées exercent une pression sur elles, cette capacité de dilatation étant négligeable.

Cette caractéristique est notamment avantageuse en ce qu'elle permet d'assurer qu'une quantité suffisante d'eau sera présente dans l'article pour permettre de remplir complètement l'enveloppe. La compresse générera ainsi du froid au maximum de sa capacité, puisque c'est la quantité d'eau absorbée par les particules qui est à la base de l'effet refroidissant : plus les particules ont absorbé d'eau, dans la limite bien sûr de leur capacité d'absorption intrinsèque, et plus l'effet de froid produit par la compresse est long et durable.

De plus, en prévoyant en outre que la quantité de particules de polymère présente dans l'enveloppe sera suffisante pour absorber toute l'eau contenue dans la poche, le fait d'introduire dans cette dernière une quantité d'eau juste égale, ou même très légèrement inférieure, au volume restant disponible dans l'enveloppe de la compresse, permet de s'assurer que toute l'eau libérée de la poche intérieure pénétrera, et sera retenue, dans l'enveloppe. Ainsi, il ne restera aucun volume d'eau libre à l'intérieur du sachet. Ceci est particulièrement avantageux, puisque l'eau restée libre dans le sachet pourrait, lors du séjour de l'article au congélateur, geler sur la surface extérieure de la compresse, et se coller sur cette dernière. Il en résulterait alors une gêne certaine pour l'utilisateur final de la compresse.

Suivant des modes de réalisation préférés dans la pratique industrielle, l'invention répond aux caractéristiques suivantes, mises en oeuvre séparément ou en chacune de leurs combinaisons techniquement opérantes.

Ces caractéristiques prennent en compte le fait que la compresse, qui est destinée à être appliquée sur la peau, et même sur des zones sensibles, telles que des plaies, doit respecter des conditions de sécurité pour l'utilisateur. Ainsi, tous ses constituants, et notamment les particules de polymère, sont non toxiques pour l'organisme.

De plus, les caractéristiques de l'article suivant l'invention permettent notamment de répondre à des objectifs supplémentaires de l'invention, qui sont d'assurer une bonne efficacité du froid délivré, ainsi qu'un bon confort d'utilisation de la compresse une fois activée.

Dans des modes de réalisation préférés de l'invention, les particules de polymère sont présentes dans l'enveloppe en un excès de 400 à 800 %, et de préférence d'environ 600 %, par rapport à la quantité qui serait juste nécessaire pour remplir l'enveloppe lorsque les particules sont complètement gonflées par absorption d'eau.

On place ainsi dans l'enveloppe une quantité de particules de polymère absorbant nettement supérieure à celle qui serait normalement requise pour que la compresse produise son plein effet refroidissant. Cette caractéristique permet avantageusement d'assurer que, même si lors du traitement de stérilisation une partie des particules est dégradée, il restera toujours dans l'enveloppe une quantité de particules opérantes, c'est-à-dire aptes à absorber une grande quantité d'eau et à la retenir pour ne la libérer qu'à l'état de vapeur, provoquant ainsi la délivrance d'un froid intense et durable par la compresse.

Dans des modes de réalisation préférés de l'invention, les particules de polymère utilisées présentent une capacité d'absorption d'eau d'environ 60 fois leur volume. Pour une enveloppe de volume interne égal à 100 ml, la quantité de particules de polymère à l'état sec contenues dans l'enveloppe est comprise entre 6 et 13 ml, de préférence environ égale à 10 ml. La poche intérieure associée contient un volume d'eau égal au complément de ce volume de particules pour arriver à 99 ml. On assure ainsi avantageusement qu'il reste suffisamment de particules de polymère opérantes après le traitement de stérilisation pour obtenir un effet refroidissant de la compresse efficace. De plus, les quatre paramètres que sont le volume interne de l'enveloppe, la capacité d'absorption des particules de polymère et leur quantité à l'état sec présente dans l'enveloppe, en prévoyant un excès important pour pallier le problème d'une dégradation partielle des particules lors de la stérilisation, et enfin le volume d'eau associé à l'intérieur du sachet extérieur, sont avantageusement choisis, en liaison les uns avec les autres, de manière à obtenir une capacité de refroidissement de la compresse maximale, en même temps qu'un bon confort d'utilisation.

Dans des modes de réalisation préférés de l'invention, le polymère est un polyacrylate de sodium réticulé. Un tel polymère n'est pas toxique pour l'organisme.

Suivant une caractéristique avantageuse de l'invention, l'enveloppe est constituée d'un non-tissé perméable à l'eau et suffisamment résistant pour ne pas céder sous l'effet des forces exercées sur lui par les particules de polymère se dilatant sous l'effet de l'absorption d'eau. Il est également avantageux dans le cadre de l'invention de prévoir que le matériau constituant l'enveloppe laisse passer l'eau, mais qu'il ne la retienne pas. Alors, les parois de l'enveloppe, et donc la surface extérieure de la compresse qui est destinée à venir en contact avec le corps, restent sèches. Il en résulte un meilleur confort pour l'utilisateur, puisque la sensation qu'il éprouve au niveau de la peau lorsque la compresse y est appliquée est celle d'un froid sec.

Dans le même objectif d'assurer un bon confort d'utilisation de la compresse, dans des modes de réalisation préférés de l'invention, le sachet extérieur est étanche à l'air et la poche d'eau et la compresse y sont conditionnées sous vide.

A cet effet, le sachet extérieur est constitué en un matériau étanche à l'air. Tout matériau présentant cette caractéristique peut être utilisé dans le cadre de l'invention, à la condition indispensable qu'il constitue également une barrière contre les germes microbiens. On préfère, pour des raisons pratiques, utiliser un sachet extérieur constitué en une matière plastique qui est telle qu'elle assure une étanchéité aux microbes, à l'air et bien sûr à l'eau afin d'éviter les risques de fuite. En outre, le sachet extérieur est fermé de manière étanche aux trois éléments que sont l'eau, les agents microbiens et l'air, notamment par des lignes de soudure continues.

Le conditionnement sous vide permet avantageusement d'éviter qu'il ne se forme une couche de givre sur la surface extérieure de la compresse lors du séjour de cette dernière au congélateur. En effet, l'air présent à l'intérieur du sachet contient toujours des traces d'humidité. Si on laisse une grande quantité d'air subsister à l'intérieur du sachet, lorsque l'article est placé au congélateur, cette humidité se condense à la surface de la compresse, et en refroidissant y forme une couche de givre. Cette couche de givre constitue une source de gêne pour l'utilisateur de la compresse.

Par contre, en appliquant une pression réduite à l'intérieur du sachet, on supprime la majeure partie de l'humidité qui y est présente, et on évite de ce fait la formation de la couche de givre. Dans la pratique, une pression correspondant à environ 95 % de vide d'air, appliquée au moyen d'appareils de mise sous pression réduite classiques, est suffisante à cet effet.

De plus, le fait de mettre l'intérieur du sachet sous pression réduite offre d'autres avantages, dus au fait que dans ces circonstances les parois du sachet extérieur se retrouvent plaquées contre les surfaces respectives de la poche d'eau et de la compresse.

D'une part on réduit ainsi le risque encouru d'ouverture du sachet extérieur lorsqu'on applique une pression sur l'article pour provoquer la rupture de la zone frangible de la poche intérieure. En effet, lorsqu'une pression réduite est appliquée à l'intérieur du sachet, les parois de ce dernier sont plaquées contre la poche d'eau : les efforts exercés sur les parois du sachet sont donc transmis directement à celles de la poche. La rupture de cette dernière est par conséquent favorisée.

D'autre part, lorsque les particules de polymère gonflent, et que la compresse augmente en épaisseur, les parois du sachet extérieur appliquées contre les parois de l'enveloppe constituent un renfort pour ces dernières, leur permettant ainsi de mieux résister aux forces exercées sur elles par les particules.

Enfin, la mise sous pression réduite a également pour effet de plaquer la poche d'eau contre la surface de la compresse, et donc de favoriser la pénétration de l'eau libérée de la poche directement dans la compresse.

L'enveloppe de la compresse suivant l'invention peut présenter différentes formes et différentes dimensions. Ainsi, il peut être décliné toute une gamme de compresses, chacune étant adaptée à une application particulière.

Dans des modes de réalisation préférés de l'invention, les faces opposées de l'enveloppe de la compresse sont reliées entre elles suivant différentes lignes longitudinales, de manière à former une pluralité de compartiments allongés dans lesquels sont réparties de manière homogène les particules de polymère. Cette forme de réalisation permet avantageusement d'obtenir une surface de contact importante de la compresse avec la peau, si bien que l'application du froid est homogène sur toute la zone de peau sur laquelle la compresse est appliquée.

Suivant une caractéristique secondaire avantageuse de l'invention, les lignes longitudinales sont réalisées par soudure par ultrasons en lignes continues ou en croisillons. Ceci permet avantageusement d'obtenir une étanchéité au passage des particules de polymère d'un compartiment à l'autre. Les lignes de soudure obtenues sont en outre mécaniquement résistantes aux efforts de traction exercées sur elles par les particules de polymère gonflant par absorption d'eau.

L'invention concerne également un procédé de fabrication de l'article pour la présentation sous forme stérile d'une compresse à base de particules de polymère à forte capacité d'absorption d'eau.

Ce procédé se caractérise par les étapes suivantes.

On prépare tout d'abord une compresse sous forme d'une enveloppe contenant les particules de polymère.

Puis on introduit la compresse, ainsi que la poche intérieure remplie d'eau, dans le sachet extérieur.

On applique une pression réduite à l'intérieur du sachet, et on ferme le sachet de façon étanche.

Enfin, on stérilise l'ensemble par traitement aux rayons gamma, à 20 à 50 kGy. La stérilisation peut également, dans des modes de réalisation préférés de l'invention, être réalisée par des rayons bêta.

Selon l'invention, la compresse est ensuite activée et utilisée de la façon suivante.

Sans ouvrir le sachet extérieur, on exerce une pression sur la poche intérieure afin de fracturer sa zone frangible. On laisse l'eau ainsi libérée de la poche intérieure pénétrer dans la compresse.

On place ensuite l'article, de façon optionnelle, au froid, notamment dans un congélateur, pendant un temps notamment compris entre 15 et 90 minutes. Cette étape permet d'augmenter la capacité refroidissante de la compresse.

Enfin, on ouvre le sachet extérieur au moment de l'utilisation pour en retirer la compresse stérile à effet refroidissant.

On peut ensuite appliquer la compresse sur la partie du corps souhaitée, en toute sécurité car elle répond aux conditions de stérilité requises.

La compresse suivant l'invention est particulièrement adaptée pour un usage unique.

L'invention sera maintenant plus complètement décrite dans le cadre de caractéristiques préférées et de leurs avantages, en faisant référence aux figures 1 à 4 dans lesquelles :
- la figure 1 représente en vue de dessus un article suivant l'invention, préalablement à l'activation de la compresse qu'il contient ;
- la figure 2 illustre l'article de la figure 1 vu en coupe suivant le plan A-A ;
- la figure 3 montre l'article de la figure 2, dans une présentation sous vide ;
- et la figure 4 représente une vue générale d'une compresse activée prête à l'emploi en sortie du sachet extérieur.

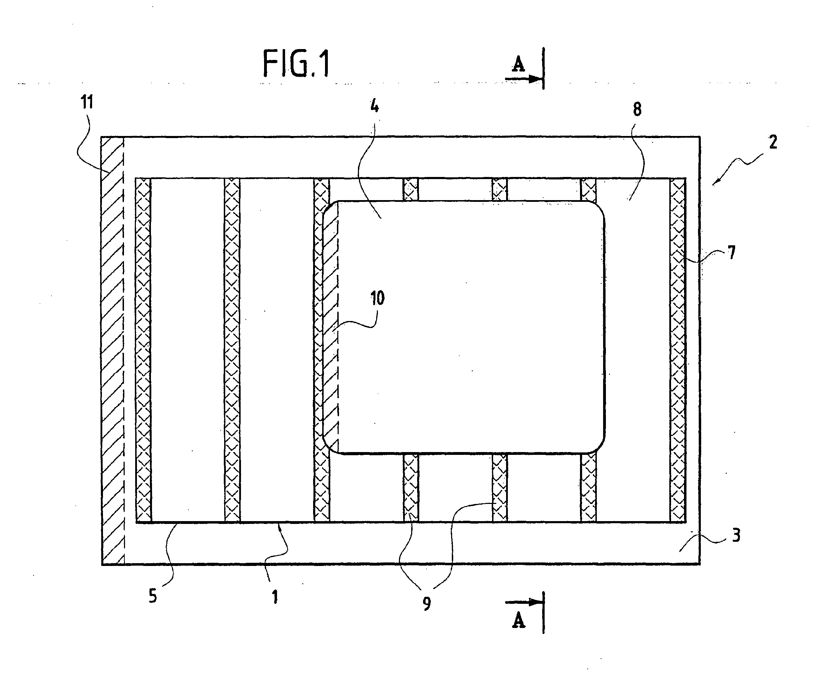

La compresse 1 suivant l'invention est présentée comme partie intégrante d'un article 2, comme illustré sur la figure 1.

L'article 2 est composé d'un sachet extérieur 3, qui est étanche à la fois à l'eau et à l'air, et qui constitue une barrière anti-microbienne. Les parois du sachet 3 sont par exemple constituées en une matière plastique constituant une barrière anti-microbienne, notamment en un mélange polyamide/polyéthylène. Un tel matériau a la particularité d'être compatible avec un traitement de stérilisation par rayons gamma. Ceci signifie que d'une part, ce matériau n'est pas dégradé lors d'un tel traitement de stérilisation, et que d'autre part il laisse passer les rayons gamma, permettant ainsi de réaliser une stérilisation efficace des éléments contenus dans le sachet extérieur 3. Il conserve ensuite les éléments qu'il contient à l'état stérile.

Dans le mode de réalisation préféré de l'invention représenté sur la figure, les parois du sachet 3 sont transparentes, si bien qu'on voit à travers elles. Ceci permet avantageusement d'être à même de surveiller l'état des éléments contenus dans le sachet 3.

A l'intérieur du sachet 3 sont disposées la compresse 1 et une poche 4 remplie d'eau.

La compresse 1 est réalisée sous forme d'une enveloppe 5 à l'intérieur de laquelle sont disposées des particules 6 d'un polymère à forte capacité d'absorption d'eau, initialement à l'état sec. Les particules 6 sont dépourvues de toxicité.

Ce sont ces particules de polymère qui sont à l'origine de l'effet de refroidissement produit par la compresse, suivant un principe général connu. Les particules 6, lorsqu'elles sont activées, c'est-à-dire lorsqu'elles sont gonflées par de l'eau qu'elles ont absorbée, et qu'elles ont éventuellement subi un séjour dans un environnement froid, génèrent du froid par vaporisation et désorption de l'eau absorbée.

Des particules de polymère 6 avantageuses dans le cadre de l'invention présentent une capacité d'absorption d'eau d'environ 60 fois leur volume. Il s'agit notamment des particules de polyacrylate réticulé, ce mode de réalisation n'étant cependant pas limitatif de l'invention.

L'enveloppe 5 qui contient les particules 6 est constituée en un matériau non-tissé perméable à l'eau. Ce matériau est compatible avec un traitement de stérilisation par rayons gamma, ce qui implique que d'une part il résiste à un tel traitement, et d'autre part il ne bloque pas le passage des rayons gamma, permettant ainsi la stérilisation des particules 6 qui se trouvent à l'intérieur de l'enveloppe.

De plus, le matériau constituant l'enveloppe présente une bonne résistance à la pression qui est exercée sur les parois de l'enveloppe par les particules de polymère lorsque celles-ci gonflent par absorption d'eau, et se dilatent donc beaucoup et de façon rapide.

L'enveloppe est notamment constituée en polypropylène.

La quantité de particules 6 à l'état sec incluse dans l'enveloppe est telle qu'il reste encore un volume libre important à l'intérieur de l'enveloppe, afin de permettre aux particules de se dilater sous l'effet de l'absorption d'eau. Ainsi, l'enveloppe se présente sous une forme quasiment plate lorsque les particules de polymère se trouvent à l'état sec, alors qu'elle présente une épaisseur qui peut être de plusieurs centimètres lorsque les particules sont gonflées d'eau.

De plus, la quantité de particules 6 à l'état sec incluse dans l'enveloppe 5 est supérieure à la quantité qui serait juste nécessaire pour remplir l'enveloppe complètement lorsque les particules sont gonflées d'eau. Elle est notamment comprise entre 400 et 800 % de cette quantité.

En d'autres termes, à titre d'exemple, pour une enveloppe de volume interne égal à 100 ml, et des particules de polymère de capacité d'absorption d'eau égale à 60 fois leur volume, si l'on introduisait 1,65 ml de particules dans l'enveloppe, une fois gonflées d'eau elles occuperaient un volume 60 fois plus important, c'est-à-dire environ égal à 100 ml. La quantité de 1,65 ml de particules à l'état sec serait donc la quantité juste nécessaire pour remplir les 100 ml de volume intérieur de l'enveloppe lorsque les particules sont complètement gonflées d'eau.

Suivant l'invention, on introduit une quantité de particules 6 de polymère quatre à huit fois plus importante, et de préférence six fois plus importante. Ainsi, toujours dans l'exemple d'une enveloppe à volume interne de 100 ml, on introduit 6 à 13 ml, de préférence environ 10 ml, de particules de polymère à l'état sec. La quantité massique de particules correspondante est aisément accessible, suivant un calcul à la portée de l'homme du métier, à partir de la masse volumique du polymère particulier choisi.

Ceci permet de façon tout à fait avantageuse d'assurer que, même si durant la stérilisation de la compresse il y a une dégradation d'une partie des particules de polymère, ou une destruction partielle de la réticulation, il reste toujours suffisamment de particules opérantes pour remplir la poche complètement après absorption d'eau, et délivrer un froid efficace.

Sur ses bords, l'enveloppe est fermée par des lignes de soudure 7, afin d'éviter que les particules 6 ne s'en échappent.

Les lignes de soudure 7 sont relativement larges, notamment de largeur comprise entre 3 et 7 mm, ce qui augmente leur résistance à l'effort exercé sur elles par les particules de polymère 6 qui se dilatent lorsqu'elles absorbent de l'eau. La technique de soudure utilisée est de préférence une soudure par ultrasons, en lignes continues ou en croisillons. Cependant, toute autre technique de soudure qui permet d'obtenir une bonne résistance de la ligne de soudure réalisée est également utilisable dans le cadre de l'invention.

L'enveloppe 5 de la compresse est de préférence réalisée de façon à former une pluralité de compartiments allongés 8, à l'intérieur desquels les particules 6 sont réparties de façon homogène. Dans l'exemple de réalisation représenté sur la figure, l'enveloppe forme six compartiments. Cette forme permet d'obtenir une surface de contact homogène de la compresse avec le corps sur lequel elle doit être appliqué, et ce sur toute la surface de la compresse.

Toute autre forme de l'enveloppe ou des compartiments entre également dans le cadre de l'invention.

Les compartiments 8 sont délimités les uns des autres par des lignes de soudure 9. Ces lignes de soudure sont réalisées de façon similaire aux lignes de soudure 7 réalisées aux extrémités de l'enveloppe 5. Il en résulte que les lignes de soudure intermédiaires 9 présentent une résistance importante aux forces qui peuvent être exercées sur elles.

On s'assure ainsi avantageusement que même si quelques unes des fibres du matériau constituant l'enveloppe sont dégradées lors du traitement de stérilisation par rayons gamma ou bêta, les lignes de soudure 7 et 9 ne seront que peu fragilisées, et qu'elles resteront suffisamment résistantes après la stérilisation pour conserver leur intégrité tout au long de l'utilisation de la compresse.

A l'intérieur du sachet extérieur 3, une poche 4 remplie d'eau est associée avec la compresse 1.

La poche 4 est constituée en un matériau étanche à l'eau et qui est également, tout comme les matériaux constituant les parois du sachet extérieur 3 et l'enveloppe 5 de la compresse, compatible avec un traitement de stérilisation par rayons gamma. Ainsi, d'une part ce matériau n'est pas dégradé par les rayons gamma, et d'autre part il ne fait pas obstacle à leur passage, si bien que l'eau contenue à l'intérieur de la poche 4 peut être stérilisée par un tel traitement.

Les parois de la poche 4 sont notamment constituées en une matière plastique. Elles sont par exemple constituées à base de polyéthylène.

La poche 4 est fermée de façon hermétique, notamment par soudure. Les soudures réalisées sont suffisamment résistantes pour ne pas céder lorsque l'article est manipulé pour son transport. Cependant, l'une au moins des soudures est conçue de telle sorte qu'elle puisse céder, au moins en partie, sous l'effet d'une pression qui serait exercée par l'utilisateur sur la surface extérieure de la poche. Par exemple, l'une au moins des soudures 10 présente une largeur inférieure à 0,5 mm, au moins dans une de ses zones.

Ainsi, lorsque l'on souhaite que l'eau contenue dans la poche 4 se répande à l'extérieur de cette poche, une pression assez forte exercée avec la main sur la poche permet de faire rompre la zone dite frangible 10 de la poche, et de libérer l'eau. Dans tous les autres cas, si des forces plus faibles sont exercées accidentellement sur la poche, il y a peu ou pas de risque qu'elle rompe.

La quantité d'eau contenue dans la poche 4 est environ égale, et de préférence légèrement inférieure, au volume qui reste libre dans l'enveloppe 5 de la compresse une fois que les particules de polymère 6 y ont été introduites. Ainsi, toujours pour un exemple d'une enveloppe 5 de volume interne égal à 100 ml, si cette enveloppe contient par exemple 10 ml de particules de polymère 6 à l'état sec, alors on lui associera une poche d'eau contenant 90 ml d'eau, ou de préférence, 89 ml. Ainsi, on s'assurera avantageusement que la totalité de l'eau contenue dans la poche 4 pourra bien pénétrer dans l'enveloppe 5, où elle sera absorbée par les particules de polymère.

Un article suivant l'invention est fabriqué de la façon suivante.

Une compresse 1 est préparée, sous forme par exemple d'un ensemble de compartiments comportant chacun une quantité adéquate de particules 6 de polymère à l'état sec.

Une poche d'eau 4 est préparée indépendamment, en prenant soin de ménager sur sa surface, et notamment au niveau d'une ligne de soudure, une zone frangible 10. La poche 4 contient uniquement la quantité d'eau adéquate, qui est déterminée en fonction d'une part du volume interne de chacun des compartiments 8, et d'autre part de la quantité de particules de polymère 6 incluse dans chacun des compartiments et de la capacité d'absorption d'eau de ces particules. Il est en outre avantageux que la poche 4 contienne le moins d'air possible.

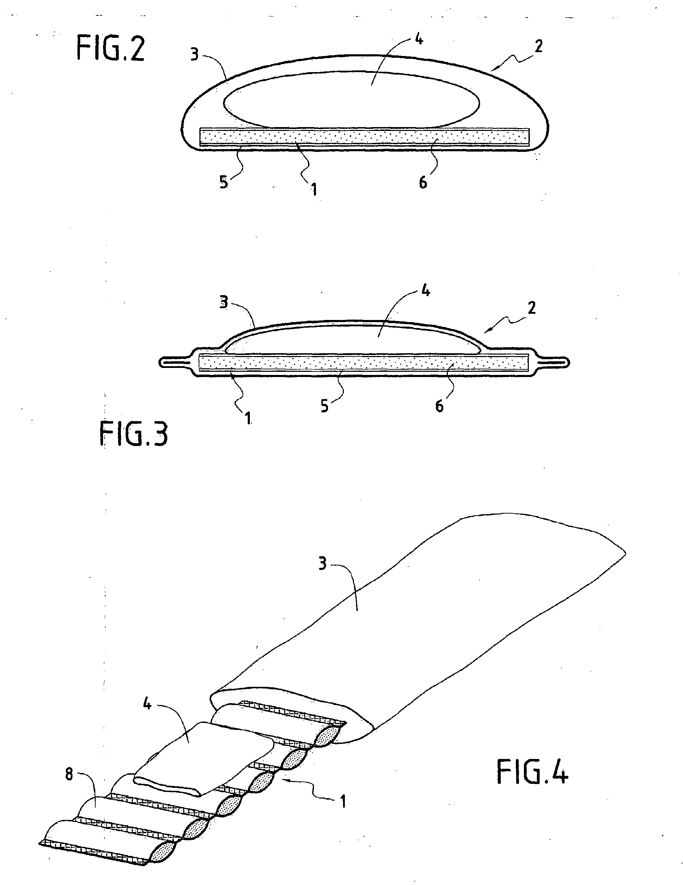

La compresse 1 et la poche 4 sont ensuite insérées dans un sachet extérieur 3 qui est suffisamment volumineux pour les contenir posées à plat l'une sur l'autre. On obtient ainsi l'article représenté en coupe suivant le plan A-A sur la figure 2.

Une pression réduite est ensuite appliquée à l'intérieur du sachet, et ce dernier est fermé. La fermeture du sachet 3 est notamment réalisée par soudure. La ligne de soudure 11 effectuée est large et résistante, de façon d'une part à garantir l'étanchéité tant à l'eau qu'à l'air du sachet, et d'autre part à assurer que lorsque l'utilisateur pressera sur la surface du sachet 3 afin de faire rompre la poche d'eau intérieure 4, ce sera bien la zone frangible 10 de cette dernière qui rompra, et non la ligne de soudure 11 fermant le sachet. Ceci est encore favorisé par le fait qu'il a été appliqué une pression réduite à l'intérieur du sachet, si bien que la pression exercée sur la surface du sachet 3 est directement transmise à celle de la poche 4. Par souci de sécurité, il est avantageux de prévoir qu'une soudure double soit effectuée pour fermer le sachet 3.

L'article se présente alors sous la configuration présentée sur la figure 3. La poche d'eau 4 est plaquée contre la surface de la compresse 1. Les parois du sachet extérieur 3 sont collées contre les surfaces de la compresse 1 et de la poche 4.

Une fois cette opération réalisée, l'article 2 est soumis à un traitement de stérilisation par rayons gamma, ou par rayons bêta. L'intérieur du sachet 3, dont la compresse 1 et l'eau de la poche 4, est alors rendu stérile. Aucun des éléments inclus dans le sachet ne subit de dommage, à l'exception d'une partie des particules 6 dont la réticulation est détruite. Il reste cependant encore suffisamment de particules opérantes pour assurer la délivrance d'un froid efficace.

Du fait des propriétés de la matière constituant les parois du sachet 3, aucun agent de contamination ne peut ensuite pénétrer à l'intérieur du sachet. L'article peut ainsi être conservé en toute sécurité, en préservant la stérilité des éléments contenus dans le sachet.

Lorsqu'on souhaite utiliser la compresse 1 pour son effet refroidissant, il suffit, sans ouvrir le sachet 3, de presser sur la surface de celui-ci, afin d'exercer une pression sur la paroi de la poche 4, et de provoquer la rupture de la zone frangible 10, et de ce fait la libération de l'eau contenue dans la poche 4.

L'eau se répand alors à l'intérieur du sachet, et elle pénètre tout naturellement à l'intérieur de la compresse 1, où elle est absorbée par les particules de polymère 6. L'eau se répartit naturellement entre les différents compartiments 8. Cependant, une répartition homogène et rapide est favorisée lorsqu'on tient l'article dans une position telle que la poche d'eau est située au-dessus de la compresse, et environ centrée sur celle-ci, au moment où l'on provoque la rupture de la poche 4.

On observe alors le gonflement des compartiments 8. Les particules en se dilatant par absorption d'eau exercent sur les parois de l'enveloppe 5 qui les contient une pression relativement forte. Les parois du sachet extérieur 3, qui sont collées contre la surface de la compresse 1, encaissent une partie de ces efforts mécaniques exercés sur l'enveloppe 5, constituant ainsi un renfort pour cette dernière, et augmentant ainsi sa résistance à ces efforts.

Lorsque l'eau qui était contenue dans la poche 4 a complètement pénétré dans la compresse, ce qui est en pratique réalisé en quelques minutes seulement, l'article est placé, toujours sans l'ouvrir, dans un environnement froid, notamment dans un congélateur. Il est maintenu dans cet endroit pendant 15 à 90 minutes, suivant la puissance du matériel de congélation utilisé, de façon à assurer le refroidissement de l'eau absorbée dans les particules de polymère, et sa transformation en glace. Ceci permettra d'obtenir une meilleure inertie de la compresse à la remontée en température lors de son utilisation.

Du fait de la pression réduite appliquée à l'intérieur du sachet, il n'y a que très peu de traces d'humidité dans l'air entourant la compresse. Il ne se forme donc pas de dépôt de givre par condensation d'humidité à la surface de la compresse durant le séjour au congélateur. La surface de la compresse reste sèche, ce qui la rendra d'autant plus confortable d'utilisation. De plus, elle ne mouillera pas la peau, et notamment la plaie sur laquelle elle sera appliquée, ce qui favorisera une cicatrisation rapide de celle-ci.

L'article 2 est ensuite retiré du congélateur. La compresse 1 présente en son intérieur est activée, c'est-à-dire qu'elle produit son effet refroidissant. Elle est entièrement stérile. Il suffit alors à l'utilisateur d'ouvrir le sachet 3, en respectant toutes les conditions de stérilité adéquates, et d'en retirer la compresse. Celle-ci se présente alors sous une forme de boudins, comme représenté sur la figure 4. Elle est prête à être appliquée sur le corps pour y fournir son effet refroidissant en toute sécurité.

Elle est particulièrement adaptée à un usage unique, et elle peut être jetée après utilisation.

A titre d'exemple, une compresse suivant l'invention est composée de particules de polyacrylate de sodium réticulé de capacité d'absorption d'eau égale à 60 fois leur volume. La compresse est composée de six boudins allongés, comme celle illustrée sur les figures. Chaque compartiment présente un volume interne de 23 ml et contient 2,7 ml de particules de polymère. La poche intérieure associée à la compresse contient 120 ml d'eau.

La compresse est préparée sous présentation stérile dans l'article suivant l'invention, et activée suivant le procédé décrit ci-avant.

Après un séjour de 90 minutes au congélateur, la compresse est extraite du sachet extérieur. Sa surface extérieure est sèche, si bien qu'elle offre un bon confort d'utilisation lorsqu'elle est appliquée sur le corps.

Elle conserve une température comprise entre 3 et 12 °C pendant 60 minutes.

Ces performances sont équivalentes à celles de compresses préparées et activées de façon classique, qui ne sont pas soumises à stérilisation préalablement à leur utilisation.

La description qui précède explique clairement comment l'invention permet d'atteindre les objectifs qu'elle s'est fixés. En particulier, elle fournit une compresse sous présentation stérile, qui présente une action refroidissante efficace et un bon confort d'utilisation.

Il ressort néanmoins de ce qui précède que l'invention n'est pas limitée aux modes de mise en oeuvre qui ont été spécifiquement décrits et représentés sur les figures et qu'elle s'étend au contraire à toute variante passant par le biais de moyens équivalents.

En particulier, la compresse qui a été décrite ci-avant présente uniquement une action refroidissante. Bien entendu, il est possible d'intégrer également dans la compresse un agent actif traitant, du type médical ou paramédical. L'agent actif peut notamment être initialement présent en solution dans l'eau contenue dans la poche intérieure, et dans ce cas il pénètre dans la compresse en même temps que l'eau. Il peut également être présent sous forme solide, à l'intérieur de l'enveloppe, en mélange avec les particules de polymère. Lorsque la compresse est appliquée sur le corps, l'agent actif y produit son effet. On obtient alors avantageusement un double effet, de refroidissement et de traitement. Des exemples d'agents actifs utilisables dans le cadre de l'invention, sous réserve qu'ils soient compatibles avec le traitement de stérilisation choisi, sont les suivants : antiseptiques, anti-inflammatoires, anesthésiants locaux, anti-oedémateux, cicatrisants.

## Revendications

1. Article comportant, à l'intérieur d'un sachet extérieur (3) étanche à l'eau, une compresse (1) à effet refroidissant réalisée sous forme d'une enveloppe (5) au moins en partie perméable à l'eau contenant des particules d'un polymère à forte capacité d'absorption d'eau à l'état sec, **caractérisé en ce que** ledit sachet (3) comporte en outre une poche intérieure (4) remplie d'eau fermée étanche à l'eau par une paroi comportant une zone frangible (10), et **en ce qu'**il est constitué en une matière de nature à maintenir ladite poche (4) et ladite compresse (1) à l'état stérile.

2. Article selon la revendication 1, **caractérisé en ce que** ladite poche intérieure (4) contient un volume d'eau sensiblement égal, de préférence légèrement inférieur, au volume disponible dans ladite enveloppe (5) pour permettre le gonflement desdites particules (6) par absorption d'eau.

3. Article selon la revendication 1 ou 2, **caractérisé en ce que** lesdites particules de polymère (6) sont présentes en un excès de 400 à 800 %, de préférence d'environ 600 %, par rapport à la quantité qui serait juste nécessaire pour remplir ladite enveloppe (5) lorsque lesdites particules (6) sont complètement gonflées par absorption d'eau.

4. Article selon l'une quelconque des revendications 1 à 3, **caractérisé en ce que** lesdites particules (6) présentent une capacité d'absorption d'eau d'environ 60 fois leur volume, et **en ce que** pour une enveloppe (5) de volume interne égal à 100 ml, la quantité de particules de polymère (6) à l'état sec contenues dans ladite enveloppe (5) est comprise entre 6 et 13 ml, de préférence environ égale 10 ml, et la poche intérieure (4) associée contient un volume d'eau égal au complément de ce volume de particules (6) pour arriver à 99 ml.

5. Article selon l'une quelconque des revendications 1 à 4, **caractérisé en ce que** ledit sachet extérieur (3) est étanche à l'air et **en ce que** ladite poche d'eau (4) et ladite compresse (1) y sont conditionnées sous vide.

6. Article selon l'une quelconque des revendications 1 à 5, **caractérisé en ce que** les faces opposées de ladite enveloppe (5) de la compresse sont reliées entre elles suivant différentes lignes longitudinales (9), de manière à former une pluralité de compartiments allongés (8) dans lesquels sont réparties de manière homogène lesdites particules de polymère (6).

7. Article selon la revendication 6, **caractérisé en ce que** lesdites lignes (9) sont réalisées par soudure par ultrasons en lignes continues ou en croisillons.

8. Article selon l'une quelconque des revendications précédentes, **caractérisé en ce que** ladite enveloppe (5) est constituée d'un non-tissé perméable à l'eau.

9. Article selon l'une quelconque des revendications précédentes, **caractérisé en ce que** ledit sachet extérieur (3), ladite poche intérieure (4) et ladite compresse (1) ont la particularité d'être compatibles avec un traitement de stérilisation par rayons gamma, notamment à 20 à 50 kGy, et/ou par rayons bêta.

10. Procédé de fabrication d'un article selon l'une quelconque des revendications 1 à 9, **caractérisé en ce que :**
- on prépare une compresse (1) sous forme d'une enveloppe (5) contenant lesdites particules de polymère (6),
- on introduit ladite compresse (1), ainsi que ladite poche intérieure (4) remplie d'eau, dans ledit sachet extérieur (3),
- on applique une pression réduite à l'intérieur dudit sachet (3), et on ferme ledit sachet (3) de façon étanche,
- et on stérilise l'ensemble par traitement aux rayons gamma, à 20 à 50 kGy, ou par rayons bêta.

11. Utilisation de l'article selon l'une quelconque des revendications 1 à 9, **caractérisée en ce que :**
- sans ouvrir ledit sachet extérieur (3), on exerce une pression sur ladite poche intérieure (4) afin de fracturer sa zone frangible (10),
- on laisse l'eau ainsi libérée de ladite poche intérieure (4) pénétrer dans ladite compresse (1),
- on place ledit article (2) optionnellement au froid, notamment dans un congélateur, pendant un temps notamment compris entre 15 et 90 minutes,
- et on ouvre ledit sachet extérieur (3) au moment de l'utilisation pour en retirer la compresse stérile à effet refroidissant (1).

## Claims

1. An item comprising, inside a watertight outer sachet (3), a compress (1) with a cooling effect produced in the form of a wrapper (5) at least partially permeable to water and containing particles of a high water absorption capacity in the dry state, wherein said sachet (3) also comprises an inner pouch (4) filled with water and sealed in a watertight manner by a wall comprising a frangible region (10), and said sachet being made of a material of a nature able to keep said pouch (4) and said compress (1) sterile.

2. The item as claimed in claim 1, wherein said inner pouch (4) contains a volume of water more or less equal to, preferably slightly less than, the volume available in said wrapper (5) to allow said particles (6) to swell by absorbing water.

3. The item as claimed in claim 1 or 2, wherein said polymer particles (6) are present in an excess of 400 to 800%, preferably of about 600%, by comparison with the quantity that would be just enough to fill said wrapper (5) when said particles (6) have swolled fully by absorbing water.

4. The item as claimed in any one of claims 1 to 3, wherein said particles (6) have a water absorption capacity of about 60 times their volume, and wherein, for a wrapper (5) with an internal volume of 100 ml, the amount of polymer particles (6) in the dry state contained in said wrapper (5) is between 6 and 13 ml, preferably about 10 ml, and the associated inner pouch (4) contains a volume of water equal to the complement to this volume of particles (6) needed to make up to 99 ml.

5. The item as claimed in any one of claims 1 to 4, wherein said outer sachet (3) is airtight and wherein said pouch of water (4) and said compress (1) are vacuum packed therein.

6. The item as claimed in any one of claims 1 to 5, wherein the opposite faces of said wrapper (5) of the compress are connected together along various longitudinal lines (9) so as to form a plurality of elongate compartments (8) in which said polymer particles (6) are uniformly distributed.

7. The item as claimed in claim 6, wherein said lines (9) are produced by ultrasonic welding in continuous lines or criss-cross lines.

8. The item as claimed in any one of the preceding claims, wherein said wrapper (5) is made of a water-permeable nonwoven.

9. The item as claimed in any one of the preceding claims, wherein said outer sachet (3), said inner pouch (4) and said compress (1) have the special feature of being compatible with a sterilization treatment using gamma-radiation, particularly at 20 to 50 kGy and/or using beta-radiation.

10. A method of manufacturing an item as claimed in any one of claims 1 to 9, wherein:
- a compress (1) in the form of a wrapper (5) containing said polymer particles (6) is prepared,
- said compress (1) and said inner pouch (4) filled with water are introduced into said outer sachet (3),
- a reduced pressure is applied to the inside of said sachet (3) and said sachet (3) is sealed in a tight manner,
- and the entity is sterilized by treating it with gamma-radiation, at 20 to 50 kGy, or with beta-radiation.

11. The use of the item as claimed in any one of claims 1 to 9, wherein:
- without opening said outer sachet (3), pressure is exerted on said inner pouch (4) in order to break its frangible region (10),
- the water thus released from said inner pouch (4) is allowed to penetrate said compress (1),
- said item (2) is optionally placed somewhere cold, particularly in a freezer, for a time of between 15 and 90 minutes for example,
- and said outer sachet (3) is opened at the time of use in order to remove the sterile cooling compress (1) therefrom.

## Patentansprüche

1. Artikel, der im Inneren eines wasserdichten äußeren Beutels (3) eine Kühlkompresse (1) aufweist, die aus einer wenigstens teilweise wasserdurchlässigen Hülle (5) besteht und Polymerpartikel mit hoher Wasseraufnahmefähigkeit im trockenen Zustand enthält, **dadurch gekennzeichnet, dass** der Beutel (3) außerdem eine mit Wasser gefüllte Innentasche (4) aufweist, die wasserdicht durch eine Wand verschlossen wird, welche einen zerbrechlichen Bereich (10) aufweist, und dadurch, dass er aus einem Material besteht, das geeignet ist, die Tasche (4) und die Kompresse (1) steril zu halten.

2. Artikel gemäß Anspruch 1, **dadurch gekennzeichnet, dass** die Innentasche (4) ein Wasservolumen enthält, das im wesentlichen gleich wie, bevorzugt etwas geringer als das in der Hülle (5) vorhandene Volumen ist, um das Quellen der Partikel (6) durch Absorption von Wasser zu ermöglichen.

3. Artikel gemäß einem der Ansprüche 1 oder 2, **dadurch gekennzeichnet, dass** die Polymerpartikel (6) in einem Überschuss von 400 bis 800%, bevorzugt von etwa 600% bezüglich der Menge vorliegen, die gerade notwendig ist, um die Hülle (5) auszufüllen, wenn die Partikel (6) durch Absorption von Wasser vollständig gequollen sind.

4. Artikel gemäß einem der Ansprüche 1 bis 3, **dadurch gekennzeichnet, dass** die Partikel (6) eine Wasseraufnahmefähigkeit aufweisen, die etwa dem 60-fachen ihres Volumens entspricht, und dadurch, dass für eine Hülle (5) mit einem Innenvolumen von 100 ml die Menge der in der Hülle (5) enthaltenen Polymerpartikel (6) im trockenen Zustand zwischen 6 und 13 ml, bevorzugt bei etwa 10 ml liegt, und die zugehörige Innentasche (4) ein Wasservolumen enthält, das der Ergänzung des Volumens der Partikel (6) auf ein Volumen von 99 ml entspricht.

5. Artikel gemäß einem der Ansprüche 1 bis 4, **dadurch gekennzeichnet, dass** der Außenbeutel (3) luftdicht ist und dass die Wassertasche (4) und die Kompresse (1) darin vakuumverpackt sind.

6. Artikel gemäß einem der Ansprüche 1 bis 5, **dadurch gekennzeichnet, dass** die gegenüberliegenden Seiten der Hülle (5) der Kompresse untereinander entlang von verschiedenen länglichen Linien (9) so verbunden sind, dass mehrere langgestreckte Kammern (8) gebildet werden, in welchen die Polymerpartikel (6) homogen verteilt sind.

7. Artikel gemäß Anspruch 6, **dadurch gekennzeichnet, dass** die Linien (9) durch Ultraschallschweißen als kontinuierliche Linien oder als gefachartige Struktur realisiert werden.

8. Artikel gemäß einem der vorhergehenden Ansprüche, **dadurch gekennzeichnet, dass** die Hülle (5) aus einem wasserdurchlässigen Fließ besteht.

9. Artikel gemäß einem der vorherigen Ansprüche, **dadurch gekennzeichnet, dass** der Außenbeutel (3), die Innentasche (4) und die Kompresse (1) die Besonderheit aufweisen, dass sie verträglich mit einer Sterilisationsbehandlung durch Gammastrahlen, insbesondere mit 20 bis 50 kGy, und/oder durch Betastrahlen sind.

10. Verfahren zur Herstellung eines Artikels gemäß einem der Ansprüche 1 bis 9, **dadurch gekennzeichnet, dass:**
- man eine Kompresse (1) in Form einer Hülle (5), welche die Polymerpartikel (6) enthält, herstellt,
- man die Kompresse (1) sowie die mit Wasser gefüllte Innentasche (4) in den Außenbeutel (3) einführt,
- man einen Unterdruck im Inneren des Beutels (3) erzeugt und den Beutel (3) dicht verschließt,
- und man die Anordnung durch Behandlung mit Gammastrahlen mit 20 bis 50 kGy oder durch Betastrahlen sterilisiert.

11. Verwendung des Artikels gemäß einem der Ansprüche 1 bis 9, **dadurch gekennzeichnet, dass:**
- man einen Druck auf die Innentasche (4) ausübt, um deren zerbrechlichen Bereich (10) zu zerbrechen, ohne den Außenbeutel (3) zu öffnen,
- man das so freigesetzte Wasser in der Innentasche (4) in die Kompresse (1) eindringen lässt,
- man den Artikel (2) während eines Zeitraums insbesondere zwischen 15 und 90 Minuten gegebenenfalls kühl lagert, insbesondere in einem Gefrierschrank,
- und man den Außenbeutel (3) zum Zeitpunkt der Benutzung öffnet, um aus ihm die sterile Kühlkompresse (1) herauszuziehen.
